**Europäisches Patentamt**

**European Patent Office**

**Office européen des brevets**

(19)

(11) Veröffentlichungsnummer: **0 271 728 B1**

# EUROPÄISCHE PATENTSCHRIFT

(12)

(45) Veröffentlichungstag der Patentschrift: **14.09.94**

(51) Int. Cl.5: **G01B 11/10**, G01N 33/36, G06K 9/46

(21) Anmeldenummer: **87116961.1**

(22) Anmeldetag: **17.11.87**

(54) **Verfahren zur Messung und/oder Überwachung von Eigenschaften von Garnen oder Seilen.**

(30) Priorität: **06.12.86 DE 3641816**

(43) Veröffentlichungstag der Anmeldung:
**22.06.88 Patentblatt 88/25**

(45) Bekanntmachung des Hinweises auf die
Patenterteilung:
**14.09.94 Patentblatt 94/37**

(84) Benannte Vertragsstaaten:
**AT BE CH DE ES FR GB IT LI LU NL SE**

(56) Entgegenhaltungen:
**EP-A- 0 100 412**
**EP-A- 0 160 895**
**DE-A- 3 129 890**
**DE-A- 3 628 654**
**FR-A- 2 141 725**

(73) Patentinhaber: **Massen, Robert, Prof. Dr.**
**Kämpfenstrasse 39**
**D-78315 Radolfzell (DE)**

(72) Erfinder: **Massen, Robert, Prof. Dr.**
**Kämpfenstrasse 39**
**D-78315 Radolfzell (DE)**

(74) Vertreter: **Leiser, Gottfried, Dipl.-Ing. et al**
**Prinz & Partner,**
**Manzingerweg 7**
**D-81241 München (DE)**

EP 0 271 728 B1

**Beschreibung**

Die Erfindung betrifft ein Verfahren zur Messung und/oder Überwachung von Eigenschaften von Garnen oder Seilen, bei welchem ein Abschnitt des Garns oder Seils durch eine Strahlenquelle beleuchtet wird und mit Hilfe eines Bildsensors, der auf die Strahlung der Strahlenquelle anspricht, ein Bild des beleuchteten Abschnitts aufgenommen und in elektrische Bildsignale umgesetzt wird, die bildpunktweise digitalisiert werden.

Nach der heute üblichen Terminologie sind unter dem Begriff "Garne" sowohl einfache Spinnfasergarne zu verstehen, die in der Spinnmaschine durch Verdrehen von Spinnfasern hergestellt werden, als auch Zwirne, die durch Verdrehen von zwei oder mehr Einfachgarnen hergestellt werden. Seile entstehen durch Drehen oder Flechten von mehreren Litzen, die ihrerseits aus mehreren Garnen gedreht oder geflochten sein können. Im Sonderfall der metallischen Seile (Drahtseile) sind die Grundbestandteile nicht Spinnfasern, sondern Drähte.

Bei der Herstellung und Verarbeitung von Garnen und Seilen müssen verschiedene Eigenschaften möglichst fortlaufend gemessen oder überwacht werden. Hierzu gehören insbesondere die folgenden Eigenschaften:

1. der Durchmesser (die Dicke) und dessen Schwankungen;
2. die Unrundheit (Abweichung von der Kreisform) und deren Schwankungen;
3. die Häufigkeit und Größe abstehender einzelner Fäden oder Drähte;
4. die mittlere Anzahl von Drehungen pro Längeneinheit sowie deren Schwankungen;
5. die Dehnung, die sich aus der Änderung der Anzahl von Drehungen pro Längeneinheit ableiten läßt.

Bei Garnen sind diese Parameter nicht nur für die Qualität der mit diesen Garnen hergestellten Textilien verantwortlich, sondern auch für einige spätere Verarbeitungsprozesse. So ist z.B. die Farbaufnahme abhängig von der Anzahl von Drehungen pro Längeneinheit. Schwankungen dieses Parameters verursachen sichtbare und störende Farbnuancen-Schwankungen, insbesondere bei Kunststoff-Garnen.

Bei Seilen ermöglicht die Überwachung dieser Parameter eine Erhöhung der Zuverlässigkeit und eine frühzeitige Erkennung von Alterungs-Prozessen. Dies ist insbesondere bei Förderanlagen mit Personenverkehr, z.B. im Bergbau, wichtig. Bei vielen technischen Garnen und Seilen muß die Dehnung kontinuierlich bestimmt werden. Die Feststellung der Häufigkeit und Größe abstehender Fäden oder Drähte ergibt einen Hinweis auf Faden- oder Drahtbrüche, die die Festigkeit beeinträchtigen.

Aus den Druckschriften EP-A-0 100 412 und DE-A-3 129 890 sind Verfahren der im Oberbegriff des Anspruchs 1 angegebenen Art bekannt, die zur Messung des Durchmessers von Garnen, Seilen oder ähnlichen Meßobjekten bestimmt sind. Bei diesen bekannten Verfahren wird ein linearer Bildsensor verwendet, der quer zur Längsrichtung des Meßobjekts orientiert ist, und das Meßobjekt ist im Strahlengang zwischen der Strahlenquelle und dem Bildsensor angeordnet, so daß die dem Bild des Meßobjekts entsprechenden Bildpunkte des vom Bildsensor aufgenommenen eindimensionalen Bildes dunkel und die übrigen Bildpunkte hell sind. Der Durchmesser des Meßobjekts ergibt sich bei Kenntnis des Abbildungsmaßstabs auf einfache Weise durch Abzählen der dunklen Bildpunkte.

Diese bekannten Verfahren sind aber nicht zur Messung der Drehung eines Garns oder Seils geeignet. Ganz allgemein ist bisher das Problem der Messung der Drehung von Garnen oder Seilen noch nicht befriedigend gelöst. Hierfür existieren rein mechanische Systeme, bei welchen eine Garnprobe eingespannt und so lange aufgedreht wird, bis die einzelnen Fasern oder Fäden parallel liegen. Auch ein bekanntes Gerät, bei welchem mit Hilfe eines punktförmigen optischen Meßkopfes die Anzahl der Drehungen pro Längeneinheit an einer Garnprobe bestimmt wird, ist nur ein Labormeßgerät für die Auswertung von kurzen Garnproben, nicht aber ein Prozeßdaten-Erfassungsgerät für den kontinuierlichen Einsatz an laufenden Garnen bei deren Fertigung oder Verarbeitung, oder gar zur Überwachung von Seilen im Betrieb. Für die Überwachung einiger Eigenschaften von metallischen Seilen existieren ferner elektromagnetische Verfahren, mit denen aber nicht alle interessierenden Parameter erfaßt werden können, und die natürlich für nichtmetallische Garne oder Seile nicht geeignet sind.

Ein weiterer Nachteil der bisher zur Messung der Drehung an laufenden Garnen oder Seilen eingesetzten Verfahren besteht darin, daß von dem laufenden Garn oder Seil ein Zeitsignal abgeleitet werden muß, so daß die Geschwindigkeit mit in das Verfahren eingeht. Die genaue Bestimmung der Geschwindigkeit ist aber mit den üblichen mechanischen Mitteln, wie z.B. einer Laufrolle, schlupffrei nicht möglich. Daher ist es mit diesen bekannten Verfahren auch nicht möglich, die Anzahl von Drehungen pro Längeneinheit und die Dehnung genau und unabhängig von der Geschwindigkeit zu bestimmen. Zur Vermeidung dieses Nachteils ist es auch bekannt, an dem Meßobjekt Markierungen anzubringen. So werden beispielsweise Schachtförderseile oft mit Zähldrähten ausgestattet, die in das Drahtseil so miteingeschlagen werden, daß sie von außen verfolgt werden können. Das Anbringen solcher Markierungen ist aber auf wenige Sonderfälle beschränkt.

Aus der EP-A-0 160 895 ist andererseits ein Verfahren zur kontinuierlichen berührungslosen Messung der Schrumpfung von Textilien bekannt, bei dem mit Hilfe von Bildsensoren, beispielsweise Fernsehkameras, das Bild eines Ausschnitts der Textiloberfläche jeweils vor und nach der Schrumpfung aufgenommen und in elektrische Bildsignale umgesetzt wird, die bildpunktweise digitalisiert und in Bildsignalspeichern abgespeichert werden. Eine Recheneinheit ermittelt aus den gespeicherten digitalen Bildsignalen die Perioden der Textilstruktur vor und nach der Schrumpfung in wenigstens einer Auswertungsrichtung, und aus dem Verhältnis der beiden in einer Auswertungsrichtung ermittelten Perioden wird die durch die Schrumpfung bewirkte Längenänderung der Textilbahn in dieser Auswertungsrichtung berechnet. Dieses Verfahren ist jedoch für die Messung der Drehung von Garnen oder Seilen weder bestimmt noch geeignet.

Aufgabe der Erfindung ist demgegenüber die Schaffung eines Verfahrens, mit welchem die Drehung von ruhenden oder bewegten Garnen oder Seilen berührungslos, zerstörungsfrei und markierungsfrei bei der Fertigung, der Verarbeitung und/oder bei der Verwendung mit großer Genauigkeit kontinuierlich gemessen bzw. überwacht werden kann.

Eine erste Lösung dieser Aufgabe besteht nach der Erfindung darin, daß ein zweidimensionales Bild des Garns oder Seils aufgenommen wird, daß die digitalen Bildsignale in einem Bildsignalspeicher an den Bildpunkten zugeordneten Speicherplätzen gespeichert werden und daß eine Recheneinheit aus den gespeicherten digitalen Bildsignalen die Periode der Struktur des Garns oder Seils in einer parallel zu dessen Längsrichtung liegenden Auswertungsrichtung ermittelt und aus der ermittelten Periode die Drehung des Garns oder Seils bestimmt.

Diese Lösung eignet sich besonders für die Messung der Drehung von Zwirnen und Seilen, da diese eine ausgeprägte periodische Struktur aufweisen.

Eine zweite Lösung der obigen Aufgabe, die besonders für die Messung der Drehung von Garnen ohne ausgeprägte periodische Struktur geeignet ist, besteht nach der Erfindung darin, daß ein zweidimensionales Bild des Garns oder Seils aufgenommen wird, daß die digitalen Bildsignale in einem Bildspeicher an den Bildpunkten zugeordneten Speicherplätzen gespeichert werden und daß eine Recheneinheit aus den gespeicherten digitalen Bildsignalen den mittleren Faserwinkel der einzelnen Fasern des Garns oder Seils ermittelt und aus dem ermittelten Faserwinkel die Drehung des Garns oder Seils bestimmt.

Da bei beiden Lösungen das Meßergebnis aus einem digital abgespeicherten zweidimensionalen Bild des Garns oder Seils gewonnen wird, spielt es keine Rolle, ob dieses Bild von einem ruhenden oder von einem mit beliebig großer Geschwindigkeit bewegten Garn oder Seil aufgenommen worden ist. Die Aufnahme des Bildes erfolgt berührungslos, ohne daß das Garn oder Seil davon in irgendeiner Weise betroffen wird. Die Auswertung der gespeicherten digitalen Bildsignale eines zweidimensionalen Bildes durch eine Recheneinheit kann in sehr kurzer Zeit erfolgen, so daß eine fortlaufende Auswertung von in kurzen Zeitabständen nacheinander aufgenommenen zweidimensionalen Bildern möglich ist. Dadurch ist eine praktisch kontinuierliche Messung und Überwachung der Drehung des Garns oder Seils möglich. Durch fortlaufenden Vergleich der aus aufeinanderfolgenden Bildern gewonnenen Meßwerte können ferner Änderungen oder Schwankungen der Drehung bestimmt werden. Infolge der Auswertung von gespeicherten digitalen Bildsignalen durch eine Recheneinheit, die vorzugsweise ein geeignet programmierter Computer ist, ist das Verfahren sehr flexibel an unterschiedliche Garne oder Seile anpaßbar. Ferner kann das Verfahren durch die Wahl der Beleuchtung, der zur Beleuchtung verwendeten Strahlung und des zur Umsetzung der Strahlung in elektrische Bildsignale verwendeten Bildsensors an die unterschiedlichsten Betriebsbedingungen optimal angepaßt werden.

Ein weiterer wesentlicher Vorteil des erfindungsgemäßen Verfahrens besteht darin, daß mit Hilfe des digital abgespeicherten Bildes ohne zusätzlichen Aufwand auch weitere Eigenschaften des Garns oder Seils gemessen oder überwacht werden können, z.B. der Durchmesser oder abstehende Fäden oder Drähte.

Die Erfindung wird mittels der folgenden Beschreibung von Ausführungsbeispielen anhand der Zeichnung näher erläufert. In der Zeichnung zeigt:

Fig. 1    das Schema einer Anordnung zur Messung und/oder Überwachung von Eigenschaften von Garnen oder Seilen,

Fig. 2    das mit dem Bildsensor der Anordnung von Fig. 1 aufgenommene Bild eines Abschnitts eines Garns oder Seils,

Fig. 3    das Diagramm einer Autokorrelationsfunktion, die bei einer Ausführungsform des Verfahrens erhalten wird, die mit der Anordnung von Fig. 1 durchführbar ist,

Fig. 4    das Diagramm einer anderen Ähnlichkeitsfunktion, die bei einer anderen Ausführungsform des mit der Anordnung von Fig. 1 durchführbaren Verfahrens erhalten wird,

Fig. 5    das Diagramm einer spektralen Transformation, die bei einer weiteren Ausführungsform des mit der Anordnung von Fig. 1 durchführbaren Verfahrens erhalten wird,

Fig. 6        das mit dem Bildsensor der Anordnung von Fig. 1 aufgenommene Bild eines Abschnitts eines Garns mit stark gestörten Faserausrichtungen ohne periodische Struktur,

Fig. 7        ein Diagramm zur Erläuterung der Bestimmung der Drehung pro Längeneinheit aus dem mittleren Faserwinkel bei einem Garn der in Fig. 6 gezeigten Art,

Fig. 8        ein Diagramm zur Erläuterung der Bestimmung des mittleren Faserwinkels durch eine Hough-Transformation,

Fig. 9        ein weiteres Diagramm zur Erläuterung der Bestimmung des mittleren Faserwinkels durch eine Hough-Transformation,

Fig. 10      ein Histogramm der ermittelten Faserwinkel mit ausgeprägtem Maximum,

Fig. 11      ein für die Auswertung ungeeignetes Histogramm ohne ausgeprägtes Maximum und

Fig. 12      das Schema einer Meßanordnung mit drei Bildsensoren zur Ermittlung der Unrundheit eines Garns oder Seils.

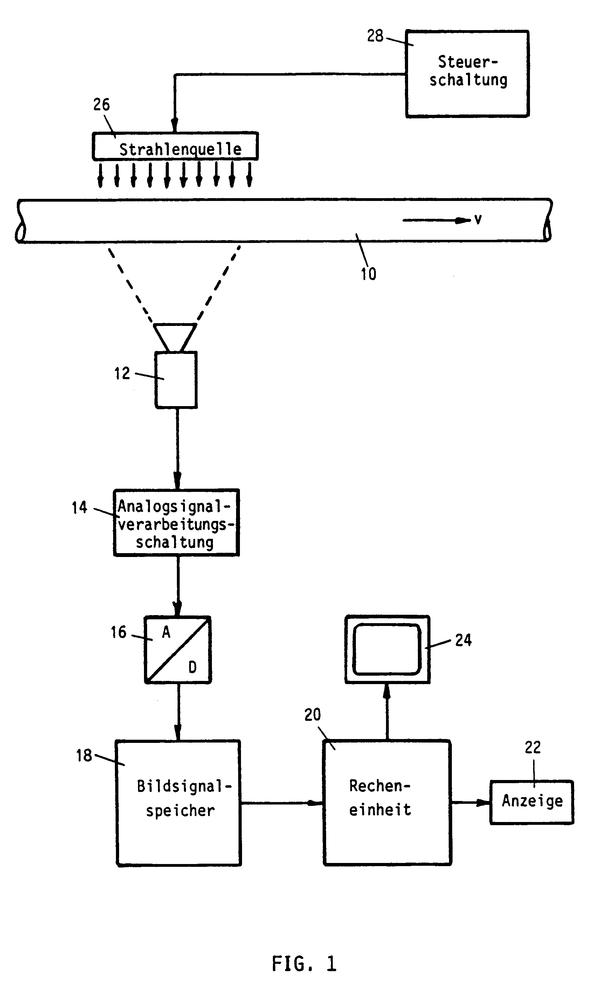

Die in Fig. 1 gezeigte Anordnung zur Messung und/oder Überwachung verschiedener Eigenschaften eines Garns oder Seils 10 enthält einen Bildsensor 12, der das Bild eines Abschnitts des Garns oder Seils 10 aufnimmt und in ein analoges elektrisches Bildsignal umsetzt, das an seinem Ausgang abgegeben wird. Der Bildsensor 12 kann eine Fernsehkamera sein, die das aufzunehmende Bild in einem genormten Fernsehraster zeilenweise abtastet, so daß das Ausgangssignal ein herkömmliches Videosignal ist. Anstelle einer Fernsehkamera können jedoch auch normfreie Matrixsensoren verwendet werden, beispielsweise CCD-Halbleiter-Matrix-Kameras. In jedem Fall muß der Bildsensor 12 in der Lage sein, ein zweidimensionales Bild des im Bildfeld liegenden Abschnitts des Garns oder Seils 10 in ein elektrisches Bildsignal umzusetzen.

Das von dem Bildsensor 12 abgegebene analoge Bildsignal wird nach der erforderlichen analogen Aufbereitung in einer Analogsignalverarbeitungsschaltung 14 einem Analog-Digital-Umsetzer 16 zugeführt, in dem es digitalisiert wird. Wenn das analoge Bildsignal ein Fernseh-Videosignal ist, entnimmt der Analog-Digital-Umsetzer 16 aus dem analogen Bildsignal periodische Abtastwerte, und er setzt jeden Abtastwert in ein digitales Signal um. Jeder Abtastwert entspricht einem Bildpunkt (Pixel) auf einer Abtastzeile des Fernsehbildes, und seine Amplitude entspricht dem Helligkeitswert (Grauwert) des Bildpunktes. Im Analog-Digital-Umsetzer 16 wird der abgetastete Amplitudenwert quantisiert und beispielsweise in eine binäre Codegruppe umgesetzt, die den Helligkeitswert in Form einer Binärzahl darstellt. Die Stellenzahl der Binärzahl, also die Bitzahl der Codegruppe, hängt von der gewünschten Quantisierungsauflösung ab.

Wenn der Bildsensor 12 eine Matrixkamera ist, kann das analoge Bildsignal bereits aus diskreten analogen Signalwerten bestehen, von denen jeder einem Bildpunkt der Matrix zugeordnet ist. Der Digital-Analog-Umsetzer setzt dann jeden analogen Signalwert unmittelbar in ein digitales Signal um, das den Helligkeitswert des betreffenden Bildpunktes darstellt.

In jedem Fall werden die digitalen Ausgangssignale des Analog-Digital-Umsetzers 16 in einen schnellen Bildsignalspeicher 18 eingegeben, der für jeden Bildpunkt eines vom Bildsensor 12 aufgenommenen zweidimensionalen Bildes einen Speicherplatz hat, an dem das vollständige digitale Signal des betreffenden Bildpunktes gespeichert werden kann.

Mit dem Leseausgang des Bildsignalspeichers 18 ist eine Recheneinheit 20 verbunden, die beispielsweise ein Mikrocomputer ist. Die Recheneinheit 20 kann die im Bildsignalspeicher 18 gespeicherten digitalen Bildsignale abrufen und daraus die Werte der zu messenden oder zu überwachenden Eigenschaften des Garns oder Seils 10 in einer später noch zu erläuternden Weise ermitteln. Die ermittelten Werte können in einer Anzeigevorrichtung 22 angezeigt oder in jeder anderen an sich bekannten Weise ausgegeben werden, beispielsweise mittels eines an die Recheneinheit 20 angeschlossenen Druckers. Ferner kann an die Recheneinheit 20 ein Monitor 24 angeschlossen sein, auf dem das aufgenommene und im Bildsignalspeicher 16 gespeicherte Bild zur Überprüfung durch eine Bedienungsperson dargestellt werden kann.

Wenn das im Bildsignalspeicher 18 gespeicherte zweidimensionale Bild von der Recheneinheit 20 vollständig ausgewertet ist, kann das nächste vom Bildsensor 12 aufgenommene Bild in digitalisierter Form in den Bildsignalspeicher 18 eingegeben und ausgewertet werden. Auf diese Weise ist eine fortlaufende Überwachung des Garns oder Seils 10 möglich. Dies ist insbesondere dann vorteilhaft, wenn das Garn oder Seil in seiner Längsrichtung an dem Bildsensor 12 vorbeibewegt wird, wie in Fig. 1 durch einen Pfeil v angedeutet ist.

Um auch bei großer Vorschubgeschwindigkeit ein scharfes Bild zu erhalten, ist es zweckmäßig, den aufzunehmenden Abschnitt des Garns oder Seils 10 durch eine starke Strahlenquelle 26 sehr kurz zu beleuchten. Eine Steuerschaltung 28 synchronisiert die Beleuchtung mit der Aufnahme durch den Bildsensor 12. Bei wiederholten Bildaufnahmen zur kontinuierlichen Messung oder Überwachung kann die kurzzeitige Beleuchtung nach Art eines Stroboskops periodisch erfolgen. Die Aufnahme kann mit sichtbarem Licht

4

erfolgen, wobei dann die Strahlenquelle 26 ein Elektronenblitz sein kann. Um von der Färbung von Garnen unabhängige Bilder der Struktur zu bekommen, kann eine Beleuchtung im Infrarotbereich zweckmäßig sein. Die üblicherweise in der Textilindustrie verwendeten Farbstoffe sind so ausgelegt, daß sie vor allem im sichtbaren Bereich des Spektrums reflektieren. Im nahen Infrarotbereich sind die Farbstoffe transparent. Es ist daher möglich, mit einer Infrarot-Beleuchtung gleichartige Bilder der Strukturen von sehr unterschiedlich gefärbten Garnen zu erhalten, und zwar von weißen über farbige bis zu tiefschwarzen Garnen.

Zur Beleuchtung können auch Wellenlängen verwendet werden, die noch weiter außerhalb des sichtbaren Spektrums liegen als der Infrarotbereich, z.B. Röntgenstrahlen zur Erzielung von Grauwert-Bildern von metallischen Seilen. Schließlich können auch andere als elektromagnetische Strahlen angewendet werden, beispielsweise Ultraschallwellen. In jedem Fall muß natürlich ein für die verwendete Strahlenart und Wellenlänge geeigneter Bildsensor verwendet werden. Zur Sichtbarmachung von Röntgenstrahlen kann man beispielsweise Videokameras in Verbindung mit Bildwandlern verwenden. Es gibt aber auch Halbleiter-Kameras, welche direkt aus der Röntgenstrahlung ein Videosignal erzeugen. Die Sichtbarmachung von Ultraschallbildern ist Stand der Technik in der Medizin.

Die Aufnahme kann im Auflicht erfolgen, so daß der Bildsensor 12 das vom Garn oder Seil reflektierte Licht empfängt, oder auch, wie in Fig. 1 dargestellt ist, im Gegenlicht, so daß das aufgenommene Bild durch die vom Garn oder Seil durchgelassene Strahlung erzeugt wird. Welche Beleuchtungsart angewendet wird, hängt von der Beschaffenheit des zu untersuchenden Garns oder Seils sowie von der angewendeten Strahlung ab.

Ein besonderer Vorteil der in Fig. 1 dargestellten Anordnung besteht darin, daß die Recheneinheit 20 aus den im Bildsignalspeicher 18 gespeicherten digitalen Bildsignalen eines einzigen zweidimensionalen Bildes gleichzeitig mehrere verschiedene Eigenschaften des Garns oder Seils erfassen kann, nämlich insbesondere:

1. den Durchmesser (die Dicke);

2. die Anzahl der Drehungen pro Längeneinheit (Meter oder Inch), bei Garnen auch "Drehungs-Zahl" oder "Drehungs-Beiwert" genannt, während bei Seilen gewöhnlich der Kehrwert, nämlich die Länge einer Drehung als "Windungshöhe" oder "Schlaglänge" angegeben wird;

3. die "Haarigkeit", d.h. die Anzahl und die Größe der von der Oberfläche abstehenden Fäden oder Drähte.

Durch Vergleich der aus mehreren nacheinander aufgenommenen Bilder eines bewegten Garns oder Seils erhaltenen Meßwerte können dann Änderungen oder Schwankungen dieser Eigenschaften festgestellt werden.

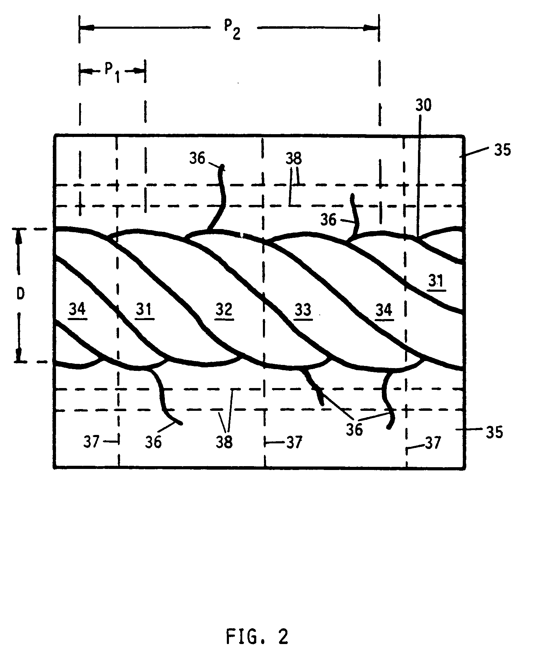

Anhand von Fig. 2, die ein mit der Anordnung von Fig. 1 aufgenommenes Bild zeigt, kann erläutert werden, wie diese verschiedenen Eigenschaften gemessen oder überwacht werden können. Die Bildfläche enthält das Bild eines Abschnitts 30 des aufgenommenen Garns oder Seils 10 und zu beiden Seiten davon ein Bild des Hintergrunds 35. Die Darstellung entspricht einem aus vier Einzelgarnen 31, 32, 33, 34 gedrehten Zwirn oder auch einem aus vier Litzen gedrehten Seil. Vor dem Hintergrund heben sich einzelne Fäden oder Drähte 36 ab, die von der Oberfläche des Garns oder Seils abstehen.

Die Messung des Durchmessers D verursacht keine besonderen Probleme. Die Recheneinheit kann entweder entlang mehreren senkrecht zur Längsachse des Garns oder Seils verlaufenden Querlinien 37 die Anzahl der zum Bild des Garns oder Seils gehörenden Bildpunkte zählen oder sie kann das Verhältnis aus der Gesamtzahl der zum Bild des Garns oder Seils gehörenden Bildpunkte zu der Gesamtzahl der Bildpunkte der aufgenommenen Bildfläche ermitteln. In beiden Fällen läßt sich der mittlere Durchmesser D unter Berücksichtigung des bekannten Abbildungsmaßstabs des Bildsensors 12 leicht berechnen. Die zum Bild des Garns oder Seils gehörenden Bildpunkte ergeben sich aus den unterschiedlichen Helligkeitswerten im Vergleich zu den Bildpunkten des Hintergrunds.

Schwieriger ist dagegen die Ermittlung der Drehung entweder als Anzahl der Drehungen pro Längeneinheit (Drehungs-Zahl, Drehungs-Beiwert) oder als axiale Länge einer Drehung (Windungshöhe, Schlaglänge). Die Anordnung von Fig. 1 bietet verschiedene Möglichkeiten zur Ermittlung der Drehung aus den gespeicherten digitalen Bildsignalen, wobei ein bevorzugtes Verfahren darin besteht, daß die periodische Struktur des Garns oder Seils ausgewertet wird. Dieses Verfahren beruht darauf, daß jedes Garn oder Seil aufgrund der Drehung seiner Bestandteile eine periodische Struktur aufweist. Diese ist bei Zwirnen oder Seilen besonders ausgeprägt. Wie die Darstellung von Fig. 2 erkennen läßt, können in diesem Fall zwei Perioden unterschieden werden:

a) eine Grundperiode $P_1$, die durch den in der Achsrichtung gemessenen Mittenabstand von zwei aufeinanderfolgenden Einzelgarnen oder Litzen bestimmt ist;

b) eine Drehungs-Periode $P_2$, die durch den Abstand von zwei aufeinanderfolgenden Windungen des gleichen Einzelgarns bzw. der gleichen Litze bestimmt ist. Die Drehungs-Periode $P_2$ ist also gleich der Windungshöhe oder Schlaglänge, deren Kehrwert gleich der Drehungszahl ist.

Zwischen den beiden Perioden $P_1$ und $P_2$ besteht ein eindeutiger Zusammenhang: Die Drehungsperiode $P_2$ entspricht dem Produkt aus der Grundperiode $P_1$ und der Anzahl der verdrehten Einzelgarne oder Litzen. Es genügt daher die Ermittlung einer dieser beiden Perioden, um den gesuchten Meßwert zu erhalten.

Die Recheneinheit 20 ermittelt die gesuchte Periode in der Längsrichtung aus den im Bildsignalspeicher 18 gespeicherten digitalen Bildsignalen durch Anwendung eines geeigneten Rechenverfahrens.

Ein bevorzugtes Verfahren zur Bestimmung der gesuchten Periode in der Längsrichtung des Garns oder Seils ist die Bildung einer eindimensionalen Ähnlichkeitsfunktion gespeicherter digitaler Bildsignale, die von wenigstens einer in der Längsrichtung verlaufenden Bildpunktreihe stammen. Eine besonders geeignete eindimensionale Ähnlichkeitsfunktion ist die Autokorrelationsfunktion. Bekanntlich ist die Autokorrelationsfunktion (abgekürzt AKF) die Korrelation einer Funktion mit der gleichen, um einen variablen Betrag verschobenen Funktion als Funktion der gegenseitigen Verschiebung. Bei dem hier vorliegenden Fall von gespeicherten Bildsignalen ist die Funktion durch die Helligkeitswerte aufeinanderfolgender Bildpunkte der ausgewerteten Bildpunktreihe bestimmt. Die Aufeinanderfolgenden Bildpunkte liegen im Bild in gleichmäßigen Abständen $\Delta x$ voneinander, die jeweils einer durch den Abbildungsmaßstab des Bildsensors bestimmten Strecke $\Delta X$ auf dem Garn oder Seil entsprechen. Die ganze Bildpunktreihe umfaßt M Bildpunkte. Wenn der erste Bildpunkt der Bildpunktreihe als Bildpunkt Nr. 1 bezeichnet wird und die Koordinate $x = 0$ hat, so hat der Bildpunkt Nr. $(i+1)$ die Koordinate $i \cdot \Delta x$, und der Bildpunkt Nr. M hat die Koordinate $(M-1) \cdot \Delta x$.

Die Autokorrelationsfunktion $R_{xx}(k)$ ist definiert durch die Summe der paarweisen Produkte der Helligkeitswerte von Bildpunkten einer Bildpunktreihe mit den Helligkeitwerten von Bildpunkten der gleichen, um k verschobenen Bildpunktreihe:

$$R_{xx}(k) = \sum_{i=0}^{L-1} I(i \cdot \Delta x) \cdot I((i+k) \cdot \Delta x) \qquad (1)$$

$$\text{mit } k = 0, 1, 2, \ldots, (Q-1)$$

Hierbei ist:

$I(i \cdot \Delta x)$: der Helligkeitwert (Grauwert) des Bildpunktes mit der Koordinate $i \cdot \Delta x$;

L: die Anzahl der für einen Wert des Verschiebeoperators k verarbeiteten Bildpunktpaare;

Q: die Anzahl der berechneten Stützwerte der Autokorrelationsfunkton.

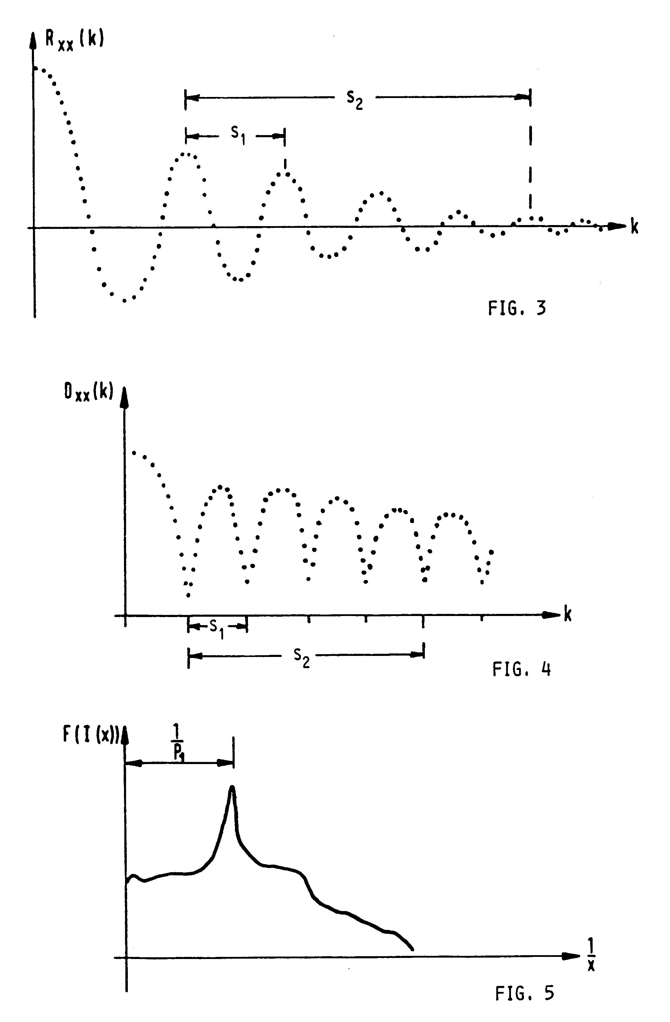

Für jeden Wert des Verschiebeoperators k erhält man nach der Gleichung (1) einen Stützwert der Autokorrelationsfunktion, insgesamt also Q Stützwerte. Somit ergibt sich beispielsweise für eine Bildpunktreihe die im Diagramm von Fig. 3 dargestellte Autokorrelationsfunktion. Die Autokorrelationsfunktion ist nicht kontinuierlich, sondern punktweise aufgebaut, wobei jeder Punkt einem Stützwert entspricht. Für bestimmte Werte von k haben die Stützwerte Maxima, weil sich bei den entsprechenden gegenseitigen Verschiebungen $k \cdot \Delta x$ infolge der Grundperiode $P_1$ der verglichenen Strukturen die maximale Übereinstimmung ergibt. Zur Vergrößerung der statistischen Genauigkeit kann man die Autokorrelationsfunktionen von mehreren ausgewählten Bildpunktreihen oder auch von allen zum abgebildeten Abschnitt des Garns oder Seils gehörenden Bildpunktreihen summieren.

Wenn der Bildsensor 12 eine Fernsehkamera ist, ist es zweckmäßig, diese so zu orientieren, daß die Achse des Bildes des Garns oder Seils parallel zur Zeilenrichtung des Fernseh-Abtastrasters liegt, weil dann die auszuwertenden Bildpunktreihen den Abtastzeilen entsprechen. Dies erleichtert die Adressierung der im Bildsignalspeicher 18 gespeicherten Bildsignale, ist aber nicht zwingend erforderlich. Ein besonderer Vorteil der Auswertung von gespeicherten Bildsignalen eines zweidimensionalen Bildes besteht gerade darin, daß die Bildsignale unabhängig von dem zeitlichen Ablauf ihrer Entstehung aufgerufen und verarbeitet werden können.

Der Abstand $S_1$ zwischen zwei aufeinanderfolgenden Maxima der Autokorrelationsfunktion im Diagramm von Fig. 3 entspricht der Grundperiode $P_1$ des in Fig. 2 dargestellten Garns oder Seils, und der Abstand $S_2$ zwischen vier aufeinanderfolgenden Maxima entspricht der Drehungs-Periode $P_2$, also unmittelbar der Windungshöhe oder Schlaglänge, deren Kehrwert die Drehungs-Zahl, also die Anzahl der Drehungen pro Längeneinheit, darstellt. Der Abstand $S_1$ entspricht einer bestimmten Anzahl $n_1$ von Bildpunktabständen $\Delta x$,

6

und der Abstand $S_2$ entspricht einer bestimmten Anzahl $n_2$ von Bildpunktabständen $\Delta x$. Infolge des durch den Abbildungsmaßstab bestimmten Zusammenhangs zwischen dem Bildpunktabstand $\Delta x$ und der entsprechenden Strecke $\Delta X$ auf dem Garn oder Seil ergibt sich die Windungshöhe oder Schlaglänge unmittelbar zu $n_2 \cdot \Delta X$.

Anstelle der Autokorrelationsfunktion können auch andere Ähnlichkeitsfunktionen zur Ermittlung der gesuchten Perioden $P_1$ bzw. $P_2$ verwendet werden. Da zur Berechnung der Autokorrelationsfunktion Produkte der gespeicherten Helligkeitswerte gebildet werden müssen, ist ein erheblicher Zeitaufwand und Hardware-Aufwand erforderlich. Eine einfacher zu berechnende Ähnlichkeitsfunktion ist die Summe der Betragsdifferenzen der gegeneinander verschobenen Helligkeitswerte:

$$D_{xx}(k) = \sum_{i=0}^{L-1} \left| I(i \cdot \Delta x) - I((i+k) \cdot \Delta x) \right| \qquad (2)$$

$$\text{mit } k = 0, 1, 2, \ldots, (Q-1)$$

Dabei haben die verschiedenen Buchstaben die zuvor bei der Gleichung (1) angegebene Bedeutung.

Die Ähnlichkeitsfunktion $D_{xx}(k)$ liefert prinzipiell die gleichen Aussagen wie die Autokorrelationsfunktion $R_{xx}(k)$, kann aber mit weniger Aufwand ermittelt werden. Fig. 4 zeigt den typischen Verlauf der Ähnlichkeitsfuktion $D_{xx}(k)$ für ein zweidimensionales Bild des Garns oder Seils. Die Grundperiode $P_1$ bzw. die der Windungshöhe oder Schlaglänge entsprechende Drehungs-Periode $P_2$ läßt sich am genauesten aus den periodischen Minima bestimmen, da diese schärfer ausgeprägt sind als die Maxima.

Die gesuchten Perioden der Struktur des Garns oder Seils lassen sich anstatt durch eine Ähnlichkeitsfunktion auch aus dem Maximum einer spektralen Transformation des digitalen Bildes bestimmen. Dies kann durch die Verwendung der Fourier-Transformation oder einer mit ihr verwandten orthogonalen Transformation, der Walsh-Transformation oder der Hadamard-Transformation geschehen. Da für zahlreiche dieser Transformationen schnelle Algorithmen bekannt sind (z.B. der Fast Fourier Transformation Algorithmus), kann durch deren Anwendung insbesondere bei der Berechnung durch Mikrocomputer eine Zeitersparnis erreicht werden. Die Grundperiode $P_1$ wird in diesem Fall aus dem dominierenden Maximum des Spektrums gewonnen, wie in Fig. 5 für die Fourier-Transformierte $F(I(x))$ dargestellt ist. Da die Autokorrelationsfunktion und die Fourier-Transformation Transformationspaare darstellen, enthalten beide die gleiche Information, allerdings in einer unterschiedlichen Darstellung.

Die Ermittlung der Drehungs-Zahl oder der Windungshöhe aus der periodischen Struktur nach einem der zuvor angegebenen Verfahren ist nicht darauf beschränkt, daß der Prüfling ein Zwirn oder Seil mit sehr ausgeprägter Struktur ist. Auch ein sogenanntes Einfachgarn oder Spinnfasergarn, das aus einer Anzahl Spinnfasern besteht, die durch Verdrehen (Spinnen) zusammengehalten sind, weist eine periodische Struktur auf, wenn die Stapellänge der verdrehten Spinnfasern größer als die Windungslänge einer Drehung ist. Die periodische Struktur entsteht dann dadurch, daß die gleichen Spinnfasern jeweils im Abstand der Windungshöhe mehrfach im Bild erscheinen. Die zuvor beschriebenen Verfahren sind für solche Periodizitäten, obwohl sie nur schwach ausgeprägt sind, sehr empfindlich. In diesem Fall fällt die Grundperiode $P_1$ mit der Drehungs-Periode $P_2$ zusammen, so daß also der Abstand zwischen zwei aufeinanderfolgenden Maxima der Autokorrelationsfunktion von Fig. 3 bzw. zwischen zwei Minima der Ähnlichkeitsfunktion von Fig. 4 unmittelbar der Windungshöhe entspricht.

Schließlich kann bei einem Zwirn oder Seil mit ausgeprägter Periodenstruktur, die auch an den Rändern der Abbildung in Erscheinung tritt (Fig. 2), die Periode auch durch Auswertung der Grauwert-Silhouette ermittelt werden, indem einfach die Maxima oder noch besser die schärfer ausgeprägten Minima der Silhouette durch Vergleich mit den Helligkeitswerten des Hintergrundes erfaßt werden und direkt der Abstand zwischen zwei Maxima oder Minima gemessen wird. Dieses Verfahren ist allerdings bei einfachen Spinnfasergarnen nicht anwendbar.

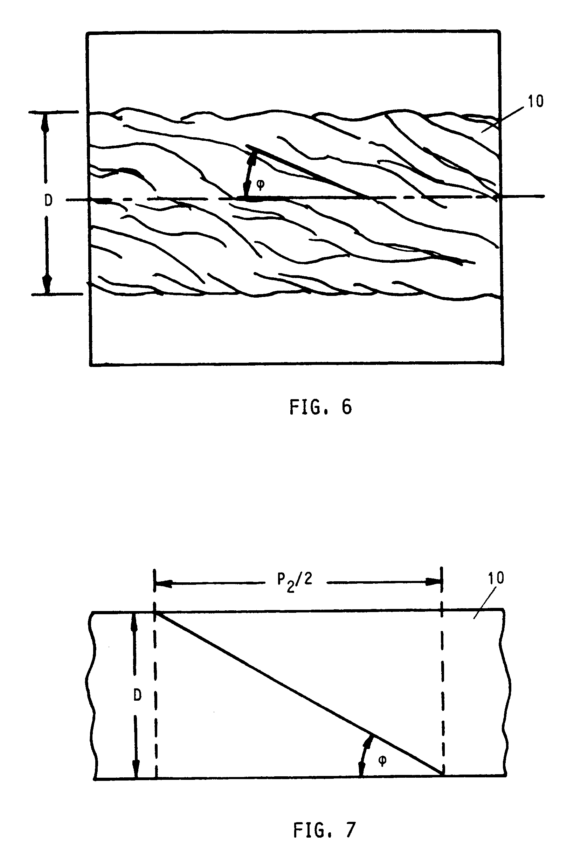

Es gibt jedoch auch aus einzelnen Spinnfasern hergestellte Garne, insbesondere im turbulenten Luftstrom rotorgesponnene Garne, bei denen die einzelnen Fasern so turbulent verwirbelt sind, daß eine periodische Drehung nicht mehr sichtbar ist. Fig. 6 zeigt das mit der Anordnung von Fig. 1 aufgenommene Bild eines solchen Garns mit stark gestörter Faserausrichtung. Bei solchen Garnen besteht eine Drehung im eigentlichen Sinne teilweise auch gar nicht mehr. Der Begriff "Drehung pro Längeneinheit" muß in diesem Fall auf einen noch meßbaren statistischen Parameter erweitert werden.

Ein noch meßbarer statistischer Parameter, der mit Hilfe der Anordnung von Fig. 1 aus den im Bildsignalspeicher 18 gespeicherten digitalen Bildsignalen ermittelt werden kann, ist der mittlere Faserwinkel $\phi$ der einzelnen Fasern, bezogen auf die Garnachse. Bei Garnen mit stark gestörten Faserausrichtungen, wie bei den im turbulenten Luftstrom rotorgesponnenen Garnen, ergibt der mittlere Faserwinkel $\phi$ eine Meßaussage, welche eine physikalisch sinnvolle Verallgemeinerung des Begriffs "Drehung pro Längeneinheit" darstellt und wie diese zur Qualitätsbeurteilung herangezogen werden kann.

Nachdem der Durchmesser D des Garns in der zuvor erläuterten Weise ermittelt worden ist, kann die "Drehungsperiode" gemäß Fig. 7 aus dem mittleren Faserwinkel $\phi$ des Garns 10 nach folgender Gleichung berechnet werden:

$$P_2 = 2 \cdot D/tg\ \phi \qquad (3)$$

Der gesuchte Faserwinkel $\phi$ kann nur aus der Oberflächenstruktur des Garns ermittelt werden, die bei seitlichem Auflicht am besten sichtbar wird. Da diese Oberflächenstruktur infolge von Unregelmäßigkeiten der Oberfläche, abstehenden Fasern, Reflexionen oder dergleichen schlecht erkennbar ist, wird das im Bildsignalspeicher 18 digital abgespeicherte Bild der Garnoberfläche zur Eliminierung oder zumindest Abschwächung dieser Störungen und zur Betonung der Kanten nach bekannten Methoden der Bildverarbeitung aufbereitet. Das so aufbereitete Bild dient dann als Grundlage für die Bestimmung des Faserwinkels.

Bei der Aufbereitung des in Form von digitalen Bildsignalen gespeicherten Bildes wird die Tatsache ausgenutzt, daß der zu erwartende Faserwinkel nur in einem bestimmten Bereich liegen kann. Deshalb können die Störungen dadurch stark verringert werden, daß die störenden Richtungen herausgefiltert werden.

Das Filtern erfolgt vorzugsweise durch Differenzieren in drei Richtungen, nämlich in der parallel zur Garnachse liegenden x-Richtung, in der senkrecht zur Garnachse liegenden y-Richtung und in der Richtung der Nebendiagonale (x-y-Richtung). Das Differenzieren in eine Richtung bewirkt, daß die in dieser Richtung verlaufenden Kanten herausgefiltert werden, während andere Kanten, vor allem die senkrecht zu dieser Richtung verlaufenden Kanten, hervorgehoben werden. Nach dem Filtern ist vorwiegend die gewünschte Richtung dominant.

Das Differenzieren wirkt auch gleichzeitig als Hochpaßfilterung, so daß feine Strukturen und Kanten hervortreten, während großflächige Strukturen und Beleuchtungsschwankungen unterdrückt oder zumindest abgeschwächt werden.

Das Differenzieren in einer Richtung erfolgt dadurch, daß das digital abgespeicherte Bild um einen Bildpunkt (1 Pixel) in der Differenzierungsrichtung verschoben wird und der Helligkeitswert jedes Bildpunkts des verschobenen Bildes von dem Helligkeitswert des zugeordneten Bildpunktes des nicht verschobenen Bildes subtrahiert wird. Das Ergebnis jeder Subtraktion ist dann der Helligkeitswert eines Bildpunktes des differenzierten und gefilterten Bildes. Bei jedem folgenden Differenzieren wird von dem durch das vorhergehende Differenzieren erhaltenen differenzierten Bild ausgegangen.

Wenn der digital abgespeicherte Helligkeitswert des Bildpunktes mit den Koordinaten x und y mit I(x,y) bezeichnet wird, wird das differenzierte und gefilterte Bild in folgenden Schritten erhalten:

1. Differenzieren in der x-Richtung:

$$I_1(x,y) = [I(x,y) - I(x+1,\ y)] + K \qquad (4)$$

Hierbei ist $I_1(x,y)$ der Helligkeitswert des Bildpunktes mit den Koordinaten x und y in dem in der x-Richtung differenzierten Bild.

2. Differenzieren in der y-Richtung:

$$I_2(x,y) = [I_1(x,y) - I_1(x,\ y+1)] + K \qquad (5)$$

Hierbei ist $I_2(x,y)$ der Helligkeitswert des Bildpunktes mit den Koordinaten x und y in dem in der x-Richtung und in der y-Richtung differenzierten Bild.

3. Differenzieren in der x-y-Richtung:

$$I_3(x,y) = [I_2(x,y) - I_2(x-1,\ y+1)] + K \qquad (6)$$

Hierbei ist $I_3(x,y)$ der Helligkeitswert des Bildpunktes mit den Koordinaten x und y in dem in allen drei Richtungen differenzierten Bild.

Der in jedem Schritt hinzuaddierte konstante Wert K sorgt dafür, daß nur positive Differenzbilder entstehen und damit eine vorzeichenlose Weiterverarbeitung erreicht wird. Um den Wertebereich der Differenzbilder zu vergrößern, können die Ausdrücke (4) bis (6) auch multiplikativ mit einem Faktor beaufschlagt werden.

Das nach dem dritten Schritt erhaltene digitale Bild dient dann als Grundlage für die Bestimmung des Faserwinkels $\phi$. Hierfür sind verschiedene Verfahren geeignet.

Einige Verfahren zur Bestimmung des Faserwinkels setzen ein binärisiertes Bild voraus, d.h. ein Bild, bei dem der Helligkeitswert jedes Bildpunktes durch ein einziges Bit dargestellt ist, das entweder den Binärwert 0 (schwarz) oder den Binärwert 1 (weiß) hat. Wenn dies erforderlich ist, wird das nach dem vorstehenden Verfahren erhaltene differenzierte und gefilterte Bild dadurch binärisiert, daß der digitale Helligkeitswert jedes Bildpunktes mit einer eingegebenen Binärisierungsschwelle verglichen wird; wenn der Helligkeitswert die Binärisierungsschwelle überschreitet, wird dem Bildpunkt der Binärwert 1 und im andern Fall der Binärwert 0 zugeordnet.

Für die Bestimmung des Faserwinkels $\phi$ haben sich insbesondere drei Verfahren als geeignet erwiesen:
- Korrelation;
- Hough-Transformation;
- Vektordrehung.

Diese drei Verfahren sollen nachstehend näher erläutert werden.

Korrelation

Für die Korrelation wird das nach dem vorstehenden Verfahren erhaltene gefilterte Bild, also ohne Binärisierung, verwendet. Dabei werden zwei Bildzeilen gegeneinander verschoben und für jeden Wert des Verschiebeoperators ein Maß für die Übereinstimmung berechnet. Von der so erhaltenen Korrelationsfunktion wird das Maximum gesucht. Aus dem Wert des Verschiebeoperators, bei dem das Maximum auftritt, und im Abstand der beiden korrelierten Bildzeilen kann der gesuchte Faserwinkel $\phi$ berechnet werden.

Je kleiner der Abstand der beiden Bildzeilen ist, um so sicherer ist die Korrelation, um so geringer ist aber andererseits die Genauigkeit des berechneten Winkels. Daher sind Zwischenwerte der erhaltenen Korrelationsfunktion erforderlich, die durch eine Spline-Interpolation gewonnen werden können.

In einem praktischen Fall werden zwei Bildzeilen mit einem Abstand von drei Bildzeilen korreliert. Zunächst wird der Mittelwert $I_m$ der Helligkeitswerte der ersten Bildzeile gebildet. Dann werden die beiden Bildzeilen innerhalb des Meßfensters jeweils um einen Bildpunkt verschoben, und für jeden Wert des Verschiebeoperators k zwischen beispielsweise 0 und 15 Bildpunkten wird ein Stützwert der Korrelationsfunktion $R_{xy}(k)$ berechnet:

$$R_{xy}(k) = \sum_{i=0}^{L-1} \left[ I_3(x_i, y) - I_m \right] \cdot \left[ I_3(x_{i+k}, y+3) - I_m \right] \quad (7)$$

Darin ist L die Anzahl der im Meßfenster liegenden Bildpunkte einer Bildzeile.

Aus den 16 Korrelations-Stützwerten wird zunächst das Maximum gesucht. Im Intervall zwischen dem Maximum und dem nächstkleineren Wert werden durch Interpolation mit einer Spline-Funktion Zwischenwerte gebildet, und mit Hilfe dieser Spline-Interpolation wird das tatsächliche Maximum in diesem Intervall ermittelt, damit der Faserwinkel $\phi$ mit ausreichender Genauigkeit erhalten wird. Aus dem Abstand $\Delta y$ der korrelierten Bildzeilen und der Verschiebung $k_{max}$, bei der das Maximum auftritt, berechnet sich dann der Faserwinkel $\phi$ nach folgender Gleichung:

$\phi = \text{arctg}(\Delta y/k_{max})$ (8)

Hough-Transformation

Die Hough-Transformation ist ein Verfahren, durch das kolineare Bildpunkte entdeckt werden. Die mathematische Grundlage bildet die Darstellung einer Geraden in der Hesse'schen Normalform:

$r = x \cdot \cos \alpha + y \cdot \sin \alpha$ (9)

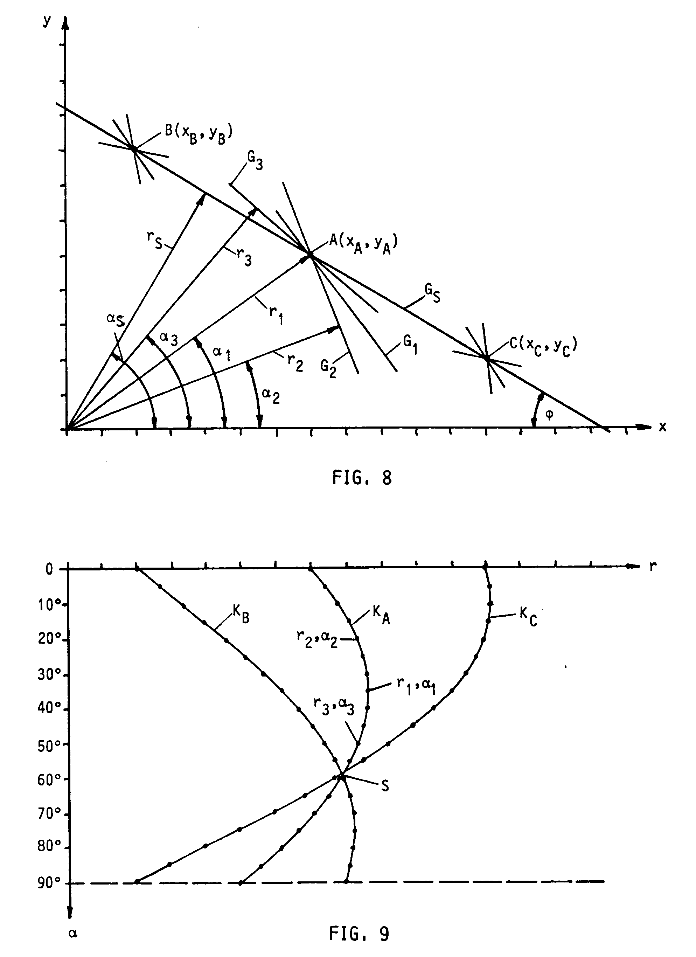

Darin ist r der Abstand des Koordinatenursprungs von der Geraden, der auf der durch den Koordinatenursprung gehenden Normalen gemessen wird, und $\alpha$ der Winkel zwischen der x-Achse und dieser Normalen. Dies ist in Fig. 8 für eine Gerade $G_1$ dargestellt, wobei der Schnittpunkt zwischen der Geraden und der durch den Ursprung gehenden Normalen mit A bezeichnet ist. Wenn die Koordinaten $x_A$, $y_A$ des Punktes A bekannt sind, können die zur Geraden $G_1$ gehörigen Konstanten $r_1$ und $\alpha_1$ der Hesse'schen Normalform leicht aus der obigen Gleichung (9) berechnet werden. Das Wertepaar $r_1, \alpha_1$ entspricht einem Punkt in einem r-$\alpha$-Diagramm, das in Fig. 9 dargestellt ist. Jeder Punkt in diesem r-$\alpha$-Diagramm entspricht einer durch die Hesse'sche Normalform eindeutig definierten Geraden im x-y-Koordinatensystem von Fig. 8. Durch einfache trigonometrische Beziehungen können für andere Geraden G, die durch den Punkt A gehen, die zugehörigen Wertepaare r,$\alpha$ berechnet und in das r-$\alpha$-Diagramm eingetragen werden. Hierzu wird beispielsweise der Winkel $\alpha$ jeweils um 5° geändert und der zugehörige Ursprungs-Abstand r berechnet. Die so berechneten Punkte liegen auf einer Kurve $K_A$, die das durch den Punkt A gehende Geradenbüschel repräsentiert. Als Beispiel sind in Fig. 8 zwei weitere Gerade $G_2$, $G_3$ dieses Geradenbüschels mit den zugehörigen Parametern $r_2$, $\alpha_2$ bzw. $r_3$, $\alpha_3$ dargestellt, und in Fig. 9 sind die zugehörigen Punkte auf der Kurve $K_A$ angegeben.

Zur Bestimmung des Faserwinkels $\phi$ werden in dem gewählten Bildausschnitt des gefilterten und binärisierten Bildes weiße Bildpunkte gesucht; für jeden dieser Punkte sind die zugehörigen Koordinatenwerte x, y bekannt. Nach dem zuvor angegebenen Verfahren können daher die Wertepaare r, $\alpha$ des durch den Bildpunkt gehenden Geradenbüschels bestimmt werden, und die Gesamtheit dieser Wertepaare kann als Kurve im r-$\alpha$-Diagramm dargestellt werden. Als Beispiel sind in Fig. 8 zusätzlich zu dem Bildpunkt A zwei weitere Bildpunkte B und C und in Fig. 9 die zugehörigen Kurven $K_B$, $K_C$ dargestellt.

Wenn sich im r-$\alpha$-Diagramm von Fig. 9 zwei Kurven schneiden, entspricht der Schnittpunkt einer Geraden, die durch die beiden zugehörigen Bildpunkte im Koordinatensystem von Fig. 8 geht. Wenn sich viele solcher Kurven im gleichen Punkt oder zumindest in einem engen Bereich schneiden, sind alle zugehörigen Bildpunkte kolinear. So entspricht der gemeinsame Schnittpunkt S im r-$\alpha$-Diagramm von Fig. 9 im Koordinatensystem von Fig. 8 der Geraden $G_S$ mit dem Wertepaar $r_S$, $\alpha_S$, die durch die drei Bildpunkte A, B und C geht.

Wenn eine solche gemeinsame Gerade $G_S$ für eine große Zahl von weißen Bildpunkten festgestellt wird, besteht eine große Wahrscheinlichkeit dafür, daß diese Bildpunkte zu derselben Faser gehören. Da die x-Richtung des Koordinatensystems der Richtung der Garnachse entspricht, ist der Winkel zwischen der Geraden $G_S$ und der x-Achse der gesuchte Faserwinkel $\phi$. Dieser Faserwinkel kann unmittelbar aus dem Diagramm von Fig. 9 entnommen werden, denn er ist der Komplementwinkel zu dem Winkel $\alpha_S$ im rechtwinkligen Dreieck:

$$\phi = 90° - \alpha_S \qquad (10)$$

Dadurch ist ein wahrscheinlicher Faserwinkel ermittelt. Der gleiche Vorgang wird für weitere Bildpunktreihen in verschiedenen Bildausschnitten wiederholt.

Vektordrehung

Voraussetzung für die Ermittlung des Faserwinkels durch Vektordrehung ist wiederum ein binärisiertes Bild. Das Prinzip besteht darin, daß ein Vektor zwischen 0° und 90° in 1°-Schritten gedreht wird und jeweils die auf ihm liegenden weißen Bildpunkte gezählt werden. Die Summe der auf dem Vektor liegenden Bildpunkte ist für jeden Winkel ein Maß für seine Ausprägung. Für jede Messung wird dieser Vorgang mehrmals wiederholt. Anschließend wird ein Histogramm der Anzahl der vorhandenen Bildpunkte für jeden Winkel ausgegeben. Über das gesamte Bild hinweg werden mehrere Messungen durchgeführt, deren Anzahl davon abhängt, wie dicht die weißen Bildpunkte in der x-Richtung liegen. Als letztes wird ein Summenhistogramm der Messungen ausgegeben. Das Maximum dieser Funktion ergibt den gesuchten mittleren Faserwinkel.

In jedem der zuvor geschilderten Fälle wird nach der Ermittlung einer möglichst großen Zahl von Faserwinkeln das Histogramm, d.h. die Häufigkeitsverteilung der ermittelten Faserwinkel gebildet. Dieses Histogramm wird geeigneten bekannten statistischen Glättungs- und Interpolationsverfahren unterzogen und stellt in seiner Gesamtheit eine repräsentative Aussage über die mittlere Drehung der versponnenen Einzelfasern und ihre Gleichmäßigkeit dar. Diese Darstellung kann auf einige Parameter des Histogramms, wie Mittelwert, häufigster Wert, Streuung der Faserwinkel usw. reduziert werden, falls der gesamte Verlauf des Histogramms zur Beurteilung nicht benötigt wird. Diese Verarbeitungsschritte können gleichzeitig mit der digitalen Bildauswertung durch den Bildrechner durchgeführt werden.

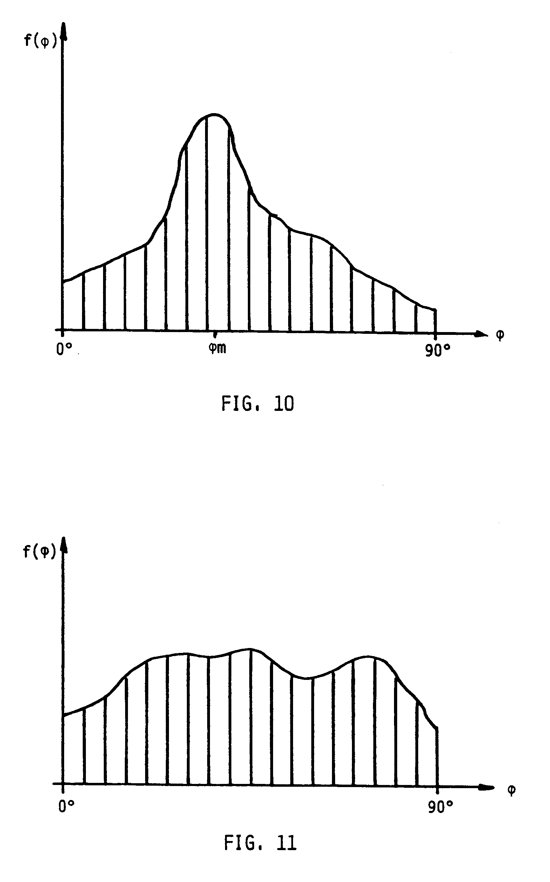

Als Beispiel ist in Fig. 10 das Histogramm der Häufigkeit f($\phi$) als Funktion der ermittelten Faserwinkel $\phi$ dargestellt. Der Winkel $\phi_m$, der mit maximaler Häufigkeit ermittelt worden ist, entspricht der dominierenden Faserrichtung.

Bei stark verwirbelten Einzelfasern gibt es lokale Stellen auf der Garnoberfläche, an denen eine solche dominierende Faserrichtung nicht vorliegt. Dies äußert sich in einem gleich verteilten Histogramm ohne typisches Maximum, wie es in Fig. 11 dargestellt ist. Durch Überwachung des Maximums des Histogramms können solche zur Messung ungeeigneten Garnstellen automatisch verworfen werden.

In Fig. 2 ist auch gezeigt, wie die Ermittlung der "Haarigkeit" durch die Recheneinheit 20 erfolgen kann. Außerhalb des Bildes des Garns oder Seils 10 werden in einem oder mehreren gedachten Prüfstreifen 38, die parallel zur Achse des Garns oder Seils verlaufen, die in den Prüfstreifen fallenden Bildpunkte von abstehenden Fäden oder Drähten aufgrund der unterschiedlichen Helligkeitswerte gegenüber den Helligkeitswerten des Hintergrunds erfaßt und gezählt. Jeder Prüfstreifen kann beispielsweise einer Bildpunktreihe oder auch mehreren nebeneinanderliegenden Bildpunktreihen entsprechen. Durch die Erfassung in mehreren parallelen Prüfstreifen kann eine Aussage nicht nur über die Häufigkeit, sondern auch über die Länge der abstehenden Fäden oder Drähte erhalten werden.

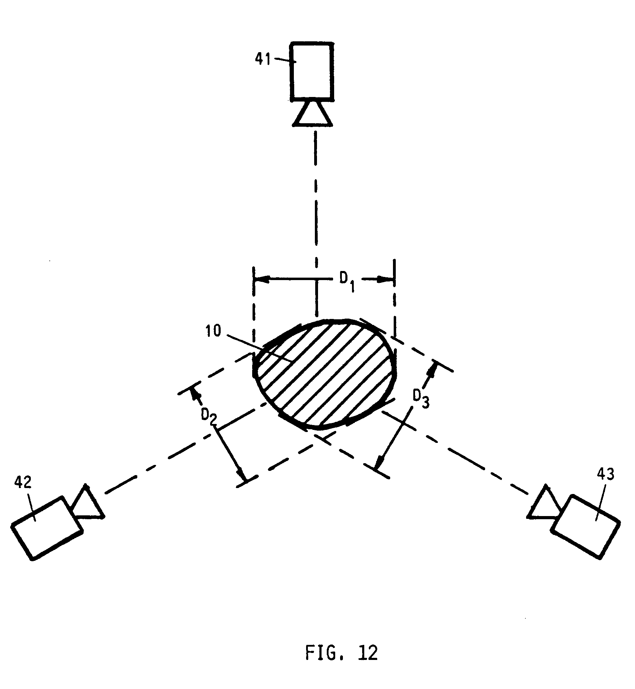

In Fig. 12 ist schließlich gezeigt, wie durch eine Anordnung von mehreren Bildsensoren die räumliche Silhouette des Garns oder Seils erfaßt werden kann und dadurch Aussagen über die dreidimensionale Geometrie, insbesondere über die Rundheit, gewonnen werden können. Das Garn oder Seil 10 ist in Fig.12 im Querschnitt gezeigt, und es sind drei Bildsensoren 41, 42, 43 mit einer gegenseitigen Winkelversetzung von 120° rings um das Garn oder Seil angeordnet. Jeder Bildsensor ist in der anhand von Fig. 1 für den Bildsensor 12 erläuterten Weise ausgebildet und mit einer Signalverarbeitungsschaltung der beschriebenen Art verbunden. Mit Hilfe der drei Bildsensoren 41, 42, 43 werden drei zweidimensionale Bilder des Garns oder Seils erhalten, und die digitalisierten Bildsignale werden gespeichert. Werden die drei Durchmesser $D_1$, $D_2$ und $D_3$ des Garns oder Seils in diesen drei Ansichten gemessen, so kann aus den Unterschieden dieser drei Durchmesser ein Maß für die Unrundheit abgeleitet werden:

$$\text{Unrundheit} = f(|D_1 - D_2|, |D_2 - D_3|, |D_3 - D_1|)$$

Es sind natürlich weitere Abänderungen und Ausgestaltungen der beschriebenen Anordnung möglich. Insbesondere können anstelle von Grauwertbildern auch Farbbilder aufgenommen, abgespeichert und ausgewertet werden. Die hierfür erforderliche Technologie ist dem Fachmann insbesondere vom Farbfernsehen geläufig. Anstelle der reinen Helligkeitswerte des Bildes werden die Intensitätswerte von drei Farbauszügen jedes Bildpunktes digitalisiert und abgespeichert. Durch geeignete Auswertung der Farbsignale, ggf. auch durch absichtliche Falschfarbenbilder, können dann entweder die zuvor angegebenen Meßwerte deutlicher erfaßt werden, oder es können noch zusätzliche Informationen über weitere Eigenschaften des Garns oder Seils gewonnen werden.

**Patentansprüche**

1. Verfahren zur Messung und/oder Überwachung der Eigenschaften von Garnen oder Seilen, bei welchem ein Abschnitt des Garns oder Seils (10) durch eine Strahlenquelle (26) beleuchtet wird und mit Hilfe eines Bildsensors (12), der auf die Strahlung der Strahlenquelle (26) anspricht, ein Bild des beleuchteten Abschnitts aufgenommen und in elektrische Bildsignale umgesetzt wird, die bildpunktweise digitalisiert werden, dadurch gekennzeichnet, daß ein zweidimensionales Bild des Garns oder Seils (10) aufgenommen wird, daß die digitalen Bildsignale in einem Bildsignalspeicher (18) an den Bildpunkten zugeordneten Speicherplätzen gespeichert werden und daß eine Recheneinheit (20) aus den gespeicherten digitalen Bildsignalen die Periode der Struktur des Garns oder Seils (10) in einer parallel zu dessen Längsrichtung liegenden Auswertungsrichtung ermittelt und aus der ermittelten Periode die Drehung des Garns oder Seils (10) bestimmt.

2. Verfahren nach Anspruch 1, dadurch gekennzeichnet, daß die Recheneinheit (20) eine eindimensionale Ähnlichkeitsfunktion gespeicherter digitaler Bildsignale berechnet, die von wenigstens einer in der Auswertungsrichtung verlaufenden Bildpunktreihe stammen, und die Periode der Struktur des Garns oder Seils (10) aus der eindimensionalen Ähnlichkeitsfunktion ermittelt.

3. Verfahren nach Anspruch 2, dadurch gekennzeichnet, daß die von der Recheneinheit (20) berechnete eindimensionale Ähnlichkeitsfunktion die Autokorrelationsfunktion ist und daß die Recheneinheit (20) die Periode der Struktur des Garns oder Seils (10) aus dem Abstand zwischen zwei oder mehr aufeinanderfolgenden Maxima der Autokorrelationsfunktion bestimmt.

4. Verfahren nach Anspruch 2, dadurch gekennzeichnet, daß die von der Recheneinheit (20) berechnete eindimensionale Ähnlichkeitsfunktion eine Differenzfunktion ist, die die Summen der Betragsdifferenzen gespeicherter digitaler Bildsignale darstellt, die von wenigstens einer in der Auswertungsrichtung verlaufenden Bildpunktreihe stammen, und daß die Recheneinheit (20) die Periode der Struktur des Garns oder Seils (10) aus dem Abstand zwischen zwei oder mehr aufeinanderfolgenden Minima der Differenzfunktion bestimmt.

5. Verfahren nach Anspruch 1, dadurch gekennzeichnet, daß die Recheneinheit (20) eine spektrale Transformation des durch die gespeicherten digitalen Bildsignale dargestellten zweidimensionalen Bildes berechnet und die Periode der Struktur des Garns oder Seils (10) aus dem dominierenden Maximum des Spektrums ermittelt.

6. Verfahren zur Messung und/oder Überwachung der Eigenschaften von Garnen oder Seilen, bei welchem ein Abschnitt des Garns oder Seils (20) durch eine Strahlenquelle (26) beleuchtet wird und mit Hilfe eines Bildsensors (12), der auf die Strahlung der Strahlenquelle (26) anspricht, ein Bild des beleuchteten Abschnitts aufgenommen und in elektrische Bildsignale umgesetzt wird, die bildpunktweise digitalisiert werden, dadurch gekennzeichnet, daß ein zweidimensionales Bild des Garns oder Seils (10) aufgenommen wird, daß die digitalen Bildsignale in einem Bildspeicher (18) an den Bildpunkten zugeordneten Speicherplätzen gespeichert werden und daß eine Recheneinheit (20) aus den gespeicherten digitalen Bildsignalen den mittleren Faserwinkel der einzelnen Fasern des Garns oder Seils (10) ermittelt und aus dem ermittelten Faserwinkel die Drehung des Garns oder Seils (10) bestimmt.

7. Verfahren nach Anspruch 6, dadurch gekennzeichnet, daß die Recheneinheit (20) die Kreuzkorrelationsfunktion gespeicherter digitaler Bildsignale berechnet, die von zwei im Abstand voneinander parallel zur Achse des Garns oder Seils (10) verlaufenden Bildpunktreihen stammen, und den mittleren Faserwinkel aus dem Maximum der Kreuzkorrelationsfunktion bestimmt.

8. Verfahren nach Anspruch 6, dadurch gekennzeichnet, daß die Recheneinheit (20) den mittleren Faserwinkel aus den gespeicherten digitalen Bildsignalen durch eine Hough-Transformation bestimmt.

9. Verfahren nach Anspruch 6, dadurch gekennzeichnet, daß die Recheneinheit (20) den mittleren Faserwinkel aus den gespeicherten digitalen Bildsignalen durch Vektordrehung bestimmt.

10. Verfahren nach Anspruch 8 oder 9, dadurch gekennzeichnet, daß die gespeicherten digitalen Bildsignale für die Bestimmung des mittleren Faserwinkels binärisiert werden.

11. Verfahren nach einem der Ansprüche 7 bis 10, dadurch gekennzeichnet, daß das digital abgespeicherte Bild vor der Bestimmung des mittleren Faserwinkels zur Kantenbetonung und Unterdrückung von Störungen aufbereitet wird.

12. Verfahren nach Anspruch 11, dadurch gekennzeichnet, daß das digital abgespeicherte Bild in mehreren Richtungen differenziert wird.

13. Verfahren nach einem der Ansprüche 1 bis 12, dadurch gekennzeichnet, daß die Recheneinheit (20) aus den gespeicherten digitalen Bildsignalen die statistischen Werte abstehender Fäden oder Drähte (36) dadurch ermittelt, daß die zu solchen abstehenden Fäden oder Drähten (36) gehörenden Bildpunkte in einem oder mehreren Prüfstreifen (38), die parallel zur Achse des Bildes des Garns oder Seils (10) verlaufen, gezählt werden.

14. Verfahren nach einem der Ansprüche 1 bis 12, dadurch gekennzeichnet, daß die Recheneinheit (20) den Durchmesser des Garns oder Seils (10) aus den gespeicherten digitalen Bildsignalen dadurch bestimmt, daß die Anzahl der zum Bild des Garns oder Seils (10) gehörenden Bildpunkte entlang einer oder mehreren quer zur Längsachse des Garns oder Seils (10) verlaufenden Linien gezählt wird.

**15.** Verfahren nach einem der Ansprüche 1 bis 12, dadurch gekennzeichnet, daß die Recheneinheit (20) den Durchmesser des Garns oder Seils (10) aus den gespeicherten digitalen Bildsignalen dadurch bestimmt, daß das Verhältnis der Gesamtzahl der zum Bild des Garns oder Seils (10) gehörenden Bildpunkte zu der Gesamtzahl der Bildpunkte der aufgenommenen Bildfläche ermittelt wird.

**16.** Verfahren nach Anspruch 14 oder 15, dadurch gekennzeichnet, daß zur Bestimmung einer Unrundheit des Garns oder Seils (10) die Durchmesser des Garns oder Seils (10) aus den gespeicherten digitalen Bildsignalen von mehreren, aus unterschiedlichen Winkeln aufgenommenen zweidimensionalen Bildern ermittelt und miteinander verglichen werden.

**Claims**

**1.** Method of measuring and/or monitoring properties of yarns or ropes in which a portion of the yarn or rope (10) is illuminated by means of a radiation source (26) and an image of the illuminated portion is taken with the aid of an image sensor (12) which responds to the radiation of the radiation source (26), which image is converted to electrical image signals that are digitized image point by image point, characterized in that a two-dimensional image of the portion of the yarn or rope (10) is taken, that the digital image signals are stored in an image signal memory (18) at memory locations associated with the image points, and in that a calculating unit (20) determines from the stored digital image signals the period of the structure of the yarn or rope (10) in an evaluation direction lying parallel to the longitudinal direction thereof and determines the twist of the yarn or rope (10) from the determined period.

**2.** Method according to claim 1, characterized in that the calculating unit (20) calculates a unidimensional similarity function of stored digital image signals which originate from at least one image point row extending in the evaluation direction, and determines the period of the structure of the yarn or rope (10) from the unidimensional similarity function.

**3.** Method according to claim 2, characterized in that the unidimensional similarity function calculated by the calculating unit (20) is the autocorrelation function, and in that the calculating unit (20) determines the period of the structure of the yarn or rope (10) from the distance between two or more consecutive maxima of the autocorrelation function.

**4.** Method according to claim 2, characterized in that the unidimensional similarity function calculated by the calculating unit (20) is a difference function representing the sums of the amount differences of stored digital image signals which originate from at least one image point row extending in the evaluation direction, and in that the calculating unit (20) determines the period of the structure of the yarn or rope (10) from the distance between two or more consecutive minima of the difference function.

**5.** Method according to claim 1, characterized in that the calculating unit (20) calculates a spectral transform of the two-dimensional image represented by the stored digital image signals and determines the period of the structure of the yarn or rope (10) from the dominant maximum of the spectrum.

**6.** Method of measuring and/or monitoring properties of yarns or ropes in which a portion of the yarn or rope (10) is illuminated by means of a radiation source (26) and an image of the illuminated portion is taken with the aid of an image sensor (12) which responds to the radiation of the radiation source (26), which image is converted to electrical image signals that are digitized image point by image point, characterized in that a two-dimensional image of the portion of the yarn or rope (10) is taken, that the digital image signals are stored in an image signal memory (18) at memory locations associated with the image points, and in that a calculating unit (20) determines from the stored digital image signals the mean fibre angle of the individual fibres of the yarn or rope (10) and determines the twist of the yarn or rope (10) from the determined fibre angle.

**7.** Method according to claim 6, characterized in that the calculating unit (20) calculates the cross-correlation function of stored digital image signals which originate from two spaced-apart image point rows extending parallel to the axis of the yarn or rope (10) and determines the mean fibre angle from the maximum of the cross-correlation function.

13

EP 0 271 728 B1

**8.** Method according to claim 6, characterized in that the calculating unit (20) determines the mean fibre angle from the stored digital image signals by a Hough transformation.

**9.** Method according to claim 6, characterized in that the calculating unit (20) determines the mean fibre angle from the stored digital image signals by a vector rotation.

**10.** Method according to claim 8 or 9, characterized in that the stored digital image signals are binarized for the determination of the mean fibre angle.

**11.** Method according to any one of claims 7 to 10, characterized in that the digitally stored image is processed for emphasizing the edges and suppressing interferences prior to the determination of the mean fibre angle.

**12.** Method according to claim 11, characterized in that the digitally stored image is differentiated in several directions.

**13.** Method according to any one of claims 1 to 12, characterized in that the calculating unit (20) determines the statistical values of projecting filaments or wires (36) from the stored digital image signals by counting the image points belonging to such projecting filaments or wires (36) in one or more test strips (38) extending parallel to the axis of the yarn or rope (10).

**14.** Method according to any one of claims 1 to 12, characterized in that the calculating unit (20) determines the diameter of the yarn or rope (10) from the stored digital image signals by counting the number of the image points belonging to the image of the yarn or rope (10) along one or more lines extending transversely of the longitudinal axis of the yarn or rope (10).

**15.** Method according to any one of claims 1 to 12, characterized in that the calculating unit (20) determines the diameter of the yarn or rope (10) from the stored digital image signals by determining the ratio of the total number of the image points belonging to the image of the yarn or rope (10) to the total number of the image points of the taken image area.

**16.** Method according to claim 14 or 15, characterized in that for determining unroundness of the yarn or rope (10) the diameters of the yarn or rope (10) are determined from the stored digital image signals of several two-dimensional images taken from different angles and said diameters are compared with each other.

**Revendications**

**1.** Procédé pour mesurer et/ou surveiller les propriétés de filés ou de cordages, dans lequel on éclaire à l'aide d'une source de rayons lumineux (26) un tronçon du fil ou du cordage (10), et, à l'aide d'un capteur d'image (12) sensible au rayonnement émis par la source de rayons lumineux (26), on enregistre une image du tronçon éclairé et on la convertit en des signaux d'image électriques, qui sont numérisés en pixels, caractérisé en ce qu'on enregistre une image bidimensionnelle du filé ou du cordage (10) ; que les signaux d'image numériques sont mémorisés dans une mémoire (18) pour signaux d'image, sur les emplacements de mémoire affectés aux pixels ; et qu'une unité de calcul (20) détermine, à partir des signaux d'image numériques mémorisés, la période de la structure du filé ou du cordage (10) dans une direction d'évaluation parallèle à sa direction longitudinale, et, à partir de cette période déterminée, la torsion du filé ou du cordage (10).

**2.** Procédé selon la revendication 1, caractérisé en ce que l'unité de calcul (20) calcule une fonction de similitude unidimensionnelle des signaux d'image numériques et mémorisés, qui proviennent d'au moins une série de pixels courant dans la direction de l'évaluation, et qui calcule la période de la structure du filé ou du cordage (10) à partir de la fonction de similitude unidimensionnelle.

**3.** Procédé selon la revendication 2, caractérisé en ce que la fonction de similitude unidimensionnelle calculée par l'unité de calcul (20) est la fonction d'autocorrélation, et que l'unité de calcul (20) détermine la période de la structure du filé ou du cordage (10) à partir de l'écart entre au moins deux maxima successifs de la fonction d'autocorrélation.

14

4. Procédé selon la revendication 2, caractérisé en ce que la fonction de similitude unidimensionnelle calculée par l'unité de calcul (20) est une fonction différentielle, qui représente les sommes des différences des valeurs des signaux d'image numériques et mémorisés qui proviennent d'au moins une série de pixels courant dans la direction de l'évaluation ; et que l'unité de calcul (20) détermine la période de la structure du filé ou du cordage (10) à partir de l'écart entre au moins deux minima successifs de la fonction différentielle.

5. Procédé selon la revendication 1, caractérisé en ce que l'unité de calcul (20) calcule une transformation spectrale de l'image bidimensionnelle représentée par les signaux d'image numériques mémorisés, et détermine la période de la structure du filé ou du cordage (10) à partir du maximum dominant du spectre.

6. Procédé pour mesurer et/ou surveiller les propriétés de filés ou de cordages, dans lequel on éclaire à l'aide d'une source de rayons lumineux (26) un tronçon du fil ou du cordage (10), et, à l'aide d'un capteur d'image (12) sensible au rayonnement émis par la source de rayons lumineux (26), on enregistre une image du tronçon éclairé et on la convertit en des signaux d'image électriques, qui sont numérisés en pixels, caractérisé en ce qu'on enregistre une image bidimensionnelle du filé ou du cordage (10) ; que les signaux d'image numériques sont mémorisés dans une mémoire (18) pour signaux d'image, sur les emplacements de mémoire affectés aux pixels ; et que l'unité de calcul (20) détermine, à partir des signaux d'image numériques mémorisés, l'angle moyen de torsion des libres individuelles du filé ou du cordage (10), et, à partir de cet angle déterminé de torsion des fibres, la torsion du filé ou du cordage (10).

7. Procédé selon la revendication 6, caractérisé en ce que l'unité de calcul (20) calcule la fonction de corrélation croisée des signaux d'image numériques mémorisés qui proviennent de deux séries de pixels, courant parallèlement à l'axe du filé ou du cordage (10) à une certaine distance l'une de l'autre, et, à partir du maximum de la fonction de corrélation croisée, détermine l'angle de torsion moyen des fibres.

8. Procédé selon la revendication 6, caractérisé en ce que l'unité de calcul (20) détermine l'angle de torsion moyen des fibres à partir des signaux d'image numériques et mémorisés, par une transformation de Hough.

9. Procédé selon la revendication 6, caractérisé en ce que l'unité de calcul (20) détermine, par rotation vectorielle, l'angle moyen de torsion des libres à partir des signaux d'image numériques mémorisés.

10. Procédé selon les revendications 8 ou 9, caractérisé en ce que les signaux d'image numériques mémorisés sont binarisés pour permettre la détermination de l'angle moyen de torsion des fibres.

11. Procédé selon l'une des revendications 7 à 10, caractérisé en ce qu'avant la détermination de l'angle moyen de torsion des libres, l'image mémorisée numériquement est traitée pour renforcer les bords et supprimer les perturbations.

12. Procédé selon la revendication 11, caractérisé en ce que l'image mémorisée numériquement est différenciée dans plusieurs directions.

13. Procédé selon l'une des revendications 1 à 12, caractérisé en ce que l'unité de calcul (20) détermine, à partir des signaux d'image numériques mémorisés, les valeurs statistiques des fils ou monofilaments écartés (36), par le fait qu'elle compte les pixels appartenant à ces fils ou monofilaments écartés (36), dans une ou plusieurs bandes d'essai (38), qui courent parallèlement à l'axe de l'image du filé ou du cordage (10).

14. Procédé selon l'une des revendications 1 à 12, caractérisé en ce que l'unité de calcul (20) détermine le diamètre du filé ou du cordage (10) à partir des signaux d'image numériques mémorisés, en comptant le nombre des pixels appartenant à l'image du filé ou du cordage (10), le long d'une ou plusieurs lignes courant perpendiculairement à l'axe longitudinal du filé ou du cordage (10).

**15.** Procédé selon l'une des revendications 1 à 12, caractérisé en ce que l'unité de calcul (20) détermine le diamètre du filé ou du cordage (10) à partir des signaux d'image numériques mémorisés, en déterminant le rapport entre le nombre total des pixels appartenant à l'image du filé ou du cordage (10) et le nombre total des pixels de la surface d'image enregistrée.

**16.** Procédé selon la revendication 14 ou 15, caractérisé en ce que, pour déterminer une ovalisation du filé ou du cordage (10), on détermine le diamètre du filé ou du cordage (10) à partir des signaux d'image numériques mémorisés provenant de plusieurs images bidimensionnelles enregistrées à partir d'angles différents, et on compare ces diamètres les uns aux autres.

FIG. 1

FIG. 2

FIG. 3

FIG. 4

FIG. 5

FIG. 6

FIG. 7

FIG. 8

FIG. 9

21

FIG. 10

FIG. 11

FIG. 12